# EUROPEAN PATENT SPECIFICATION

(11) **EP 3 914 258 B1**
(45) Date of publication and mention of the grant of the patent: **29.11.2023**
(21) Application number: 20705517.9
(22) Date of filing: 24.01.2020
(51) Int. Cl.: A61K 31/7088, A61K 31/728, A61K 8/00, A61K 9/00, A61K 36/00, A61K 47/00, A61K 48/00, A61P 1/02, C12N 15/00, A61K 8/73, A61K 47/26, A61Q 11/00, A61K 8/64

(54) **A COMPOSITION FOR THE TREATMENT OF PERIODONTITIS AND REGENERATION OF INTERDENTAL PAPILLA**
ZUSAMMENSETZUNG ZUR BEHANDLUNG VON PERIODONTITIS UND REGENERATION VON INTERDENTALPAPILLEN
COMPOSITION POUR LE TRAITEMENT DE LA PARODONTITE ET LA RÉGÉNÉRATION DE LA PAPILLE INTERDENTAIRE

(30) Priority: 24.01.2019 IT 201900001081
(43) Date of publication of application: 01.12.2021
(73) Proprietor: Mastelli S.r.l., 18038 Sanremo (IM) (IT)
(72) Inventor: PRUSSIA, Giovanni, 18038 Sanremo (Imperia) (IT); PRUSSIA, Claudia, 18038 Sanremo (Imperia) (IT); CATTARINI MASTELLI, Giulia, 18038 Sanremo (Imperia) (IT)
(74) Representative: Tagliafico, Giulia
(86) International application number: PCT/IB2020/050567
(87) International publication number: WO 2020/152643

(56) References cited:
- EP-A2- 2 407 147
- JP-A- 2018 197 196
- US-A1- 2018 325 798
- KRISTINA BERTL ET AL: "Can hyaluronan injections augment deficient papillae at implant-supported crowns in the anterior maxilla? A randomized controlled clinical trial with 6 months follow-up", CLINICAL ORAL IMPLANTS RESEARCH, vol. 28, no. 9, 1 September 2017 (2017-09-01), pages 1054-1061, XP055628655, DK ISSN: 0905-7161, DOI: 10.1111/clr.12917 cited in the application
- WILLIAM BECKER ET AL: "Minimally Invasive Treatment for Papillae Deficiencies in the Esthetic Zone: A Pilot Study", CLINICAL IMPLANT DENTISTRY AND RELATED RESEARCH, vol. 12, no. 1, 1 March 2010 (2010-03-01), pages 1-8, XP055628697, CA ISSN: 1523-0899, DOI: 10.1111/j.1708-8208.2009.00247.x
- ALESSANDRA BITTO ET AL: "Adenosine receptor stimulation by polynucleotides (PDRN) reduces inflammation in experimental periodontitis", JOURNAL OF CLINICAL PERIODONTOLOGY, vol. 40, no. 1, 3 October 2012 (2012-10-03), pages 26-32, XP055628930, DK ISSN: 0303-6979, DOI: 10.1111/jcpe.12010
- STEFANO GUIZZARDI ET AL: "Hyaluronate Increases Polynucleotides Effect on Human Cultured Fibroblasts", JOURNAL OF COSMETICS, DERMATOLOGICAL SCIENCES AND APPLICATIONS, vol. 03, no. 01, 1 January 2013 (2013-01-01), pages 124-128, XP055451152, ISSN: 2161-4105, DOI: 10.4236/jcdsa.2013.31019

## Description

The present invention refers to a composition for use in the treatment of periodontitis and the regeneration of the interdental papilla,

Any reference to methods of treatment in this description is to be interpreted as a reference to the compounds, pharmaceutical compositions and medicaments of the present invention for use in a method for treatment of the human (or animal) body.

Periodontitis is a disease characterised by inflammation of the periodontal tissues. It is one of the most prevalent chronic inflammatory diseases in the population of Western countries: in Italy, it is estimated to affect, in its severe form, about 10-15% of adults, while a percentage between 2 and 30% is estimated to be affected by a mild form thereof. Periodontitis is the main cause of dental element loss; it is responsible for a serious functional deficit and at the same time can negatively affect relationships, with a significant impact on the psychological sphere, compromising the smile aesthetics.

The characteristic sign of periodontitis is the loss of attachment of the teeth to the alveoli, which results in formation of periodontal pockets, loose teeth, bleeding gums, abscesses and suppurations, ending in the loss of one or more teeth.

Conventionally, treatment of periodontitis first of all involves controlling bacteria and inflammation, and immediately afterwards, the subsequent step of regenerating the lost, hard and soft periodontal tissues becomes essential, in order to restore tooth attachment and eliminate gingival and bone pockets.

The interdental papilla is the portion of the free gingiva that occupies the space between two adjacent teeth and causes the free gingival margin to run in the dental arches with a festooned pattern. The papilla, in addition to having significance in the biology and coating of the proximal portion of the teeth, has an important aesthetic significance especially in the front regions. Explicit attention to the preservation of all interproximal tissues for aesthetic purposes began with the 1985 article by Evian and co-workers (Evian CI, Corn H, Rosenberg ES. Retained interdental papilla procedure for maintaining anterior esthetics. Compend Contin Educ Dent 1985;6(1):58-64), which describes a surgical method with specific aesthetic purposes, defining it as a "retained interdental papilla procedure".

The loss of this portion of gingival tissue is also the subject of studies, in particular with reference to possible conservation and regeneration techniques. Dental aesthetics, in fact, has become increasingly of paramount importance, both for dentists and patients. The presence of interproximal papillae between the anterior maxillary teeth is, in fact, a key aesthetic component. The recession of the interdental papilla causes a number of functional problems, as well as aesthetic problems, such as the formation of the so-called black triangle ("black triangle syndrome" (or "recession of the interincisal papilla").

In 1998, Nordland and Tarnow classified the loss of papillary height using a scale, which is still widely used and identifies four classes, namely Normal, Class I, Class II and Class III (Nordland WP, Tarnow DP. A classification sistem for loss of papillary heiht. J Periodontol 1998;69:1124-6). The classes identified by Nordland and Tarnow use the following anatomical references:
1) interdental contact point;
2) apical/facial extension of the CEJ (Cementum Enamel Junction);
3) coronal/proximal extension of the CEJ.

These classes are briefly described below:
- Normal: the papilla fills the interdental space up to the contact point.
- Class I: the tip of the papilla is located between the contact point and the coronal part of the CEJ (the space is present but the coronal/proximal CEJ is not visible).
- Class II: the tip of the papilla is located apical to the coronal/proximal CEJ, but level with the facial CEJ (visible coronal/proximal CEJ).
- Class III: the tip of the papilla is located apical to the facial CEJ.

When interdental tissue loss has already occurred, numerous techniques with reconstructive purposes can be implemented. Methods proposed in the literature suitable for solving this tissue deficiency can be divided, in all, into surgical and non-surgical techniques, even though this distinction is merely descriptive since, frequently, surgical and non-surgical techniques can be combined with each other.

### Surgical reconstruction techniques

Numerous surgical stratagems have been described in the literature with the purpose of correcting interdental tissue deficiency. However, the cited knowledge is often limited to case studies.

In 1992, Beagle proposed a surgical method, called the pedunculated flap technique, in order to recreate the interdental papilla between the upper central incisors (Beagle JR. Surgical reconstruction of the interdental papilla: case report. Int J Periodontics Restorative Dent 1992;12(2):145-51). The applicability of this technique is limited to large interdental areas and possibly facilitated by thick biotypes due to a greater presence of connective tissue.

In 1994, Aubert and co-workers proposed the deep rotated connective tissue flap method, seeking greater conservation of the nutritional supply to the tissues involved (Aubert H, Bertrand G, Orlando S, Benguigui F, Acocella G. Lembo connettivale profondo di rotazione per ricostruire la papilla interdentale. Minerva Stomatologica 1994;43 (7-8):351-357). However, this technique has the disadvantage that it can be used for one papilla at a time.

Still based on the intuition of the usefulness of a connective tissue filler, but with a view of simplifying the surgical technicality, in 1996 Han and Takei proposed the semilunar coronally repositioned papilla technique (Han TJ, Takei HH. Progress in gingival papilla reconstruction. Periodontol 2000 1996;11:65-8). This method is inspired by the consolidated reliability of the bilaminar technique, consisting in the use of a connective tissue graft underneath a partial thickness flap. One of the advantages of this technique is the dual blood supply from both the connective tissue and the overlying flap, which favours graft survival (Langer B, Langer L. Subepithelial connective tissue graft technique for root coverage. J Periodontol 1985;56(12):715-20). Despite the intuition that the use of a bilaminar technicality leads to practical advantages, compared to the method previously proposed by Aubert et al., 1994, the coronal shift of the interdental tissue complex, introduced by Han and Takei, still represents an actual technical difficulty.

In 1998, Azzi et al. proposed the envelope technique, an approach similar to that previously described, but with some changes that potentially improve its applicability (Azzi R, Etienne D, Carranza F. Surgical reconstruction of the interdental papilla. Int J Periodontics Restorative Dent. 1998;18(5):466-73).

Finally, the therapeutic difficulties associated with a significant tissue deficiency and the search for long-term stability led Azzi et al. to propose, in 2001, the use of an autologous bone graft (Azzi R, Takei HH, Etienne D, Carranza FA. Root coverage and papilla reconstruction using autogenous osseous and connective tissue grafts. Int J Periodontics Restorative Dent 2001;21(2):141-7).

In general, the aforementioned surgical reconstruction therapies create an aesthetic-anatomical compromise, altering the physiological relationship between soft and hard periodontal tissues. The clinical consequence is that a periodontal pocket or, at best, a long junctional epithelium is formed between the dental elements and the new interdental tissue. At the same time, the disruption of the normal relationship between the tissues leads to high uncertainty of the outcome in the long term.

### Non-surgical reconstruction techniques

Non-surgical reconstruction techniques are basically aimed at modifying non-gingival anatomical determinants related to the presence of interdental tissues. Therefore, their use makes it possible to act on the interdental space and/or on the distance between the contact point and the bone crest. Three types of approaches are suggested for these techniques:
- Restorative/prosthetic
- Orthodontic
- With curettage and injection of fibroblasts

The potential of the restorative/prosthetic treatment consists in modifying the coronal morphology of the tooth; hence the possibility of creating more apical contact points, inducing dental diastema closure, or reducing interdental spaces (Prato GP, Rotundo R, Cortellini P, Tinti C, Azzi R. Interdental papilla management: a review and classification of the therapeutic approaches. Int J Periodontics Restorative Dent 2004;24(3):246-55). The possibility of recreating new and more favourable anatomical determinants, with the aim of inducing proliferative responses of soft tissues, also leads to aesthetic closure of the so-called black triangles. Importantly, the extent of the morphological modification of the tooth should be evaluated on the basis of the expected response of the soft tissues. It is therefore not considered a good practice to correct papilla-free interdental areas with a total reconstructive closure of the tooth. The resulting overcorrection, in fact, can induce unwanted tissue reactions.

Orthodontic treatment also modifies the anatomical determinants, but in this case, this is carried out with approaches that are altogether more physiological. Periodontal disease, together with problems of bone resorption and soft tissue recession, often leads to tooth displacement, especially in the anterior-superior area. When the support tissue is reduced and inflamed, the dental elements respond to functional forces with migration, inclination and flaring movements. This results in accentuation of interproximal tissue loss due to changes in the relationships between the anatomical determinants. After appropriate periodontal therapy, orthodontic treatment can therefore be useful for corrective purposes (Cardaropoli D, Re S, Corrente G, Abundo R. Reconstruction of the maxillary midline papilla following a combined orthodontic-periodontic treatment in adult periodontal patients. J Clin Periodontol 2004;31(2):79-84; Cardaropoli D, Re S. Interdental papilla augmentation procedure following orthodontic treatment in a periodontal patient. J Periodontol 2005;76(4):655-61; Melsen B, Agerbaek N, Markenstam G. Intrusion of incisors in adult patients with marginal bone loss. Am J Orthod Dentofacial Orthop 1989;96(3):232-41). However, even orthodontic therapy has application limits. Regardless of the possible anchoring difficulties, obvious limitations, in fact, derive from the interarch relationships. For this reason, orthodontic therapy is often assisted, in the final stage, by conservative-prosthetic corrections.

In 1985, Shapiro described a procedure of periodic curettage of papillary tissues to stimulate their regrowth (Shapiro A. Regeneration of interdental papillae using periodic curettage. Int J Periodontics Restorative Dent 1985;5(5):26-33). This simple manoeuvre, repeated every 15 days for 3 months, has proven to be capable of inducing soft tissue hyperplasia. However, this technique can be a plausible option only in the case of very aggressive periodontal disease, and anyway, positive results have not been obtained in all cases treated by the author.

In 2007, the proposal of injection of fibroblasts into interdental tissues was published (McGuire MK, Scheyer ET. A randomized, double-blind, placebo-controlled study to determine the safety and efficacy of cultured and expanded autologous fibroblast injections for the treatment of interdental papillary insufficiency associated with the papilla priming procedure. J Periodontol 2007;78(1):4-17). The authors described a non-surgical method represented by the injection of successive quantities of autologous fibroblasts (FBL). The basis for this technicality lies in the trophic role attributed to these cells. FBLs, by producing collagen matrix and fibres, play an important role in healing and maintaining the health of the gum. It was therefore hypothesized that papilla regrowth could be promoted by using these cells. However, the peculiarity of the proposed technicality limits its applicability on an outpatient basis.

More recently, in addition to traditional therapies, less invasive treatments were proposed by infiltration of hyaluronic acid into the gum. Treatments are carried out by filling in the missing papillary tissue with fillers. This filling is carried out by using cross-linked hyaluronic acid, which acts as a filler, in order to create a volume and fill in the missing area of the papilla (Bertl K, Gotfredsen K, Jensen SS, Bruckmann C, Stavropoulos A. Can hyaluronan injections augment deficient papillae at implant-supported crowns in the anterior maxilla? A randomized controlled clinical trial with 6 months follow-up. Clin Oral Implants Res. 2017 Sep;28(9):1054-1061(a)). Cross-linked hyaluronic acid, which creates a volumetric effect in the tissue where it is infiltrated, can even persist for many months, since degradation occurs very slowly. In fact, the molecule is protected from hyaluronidase degradation as a result of the chemical modification treatment performed by means of the cross-linking step. However, the results obtained are conflicting and not without the occurrence of adverse reactions (Bertl K, Gotfredsen K, Jensen SS, Bruckmann C, Stavropoulos A. Adverse reaction after hyaluronan injection for minimally invasive papilla volume augmentation. A report on two cases. Clin Oral Implants Res. 2017 Jul;28(7):871-876)(b)).

Prior art document W. Becker (CLINICAL IMPLANT DENTISTRY AND RELATED RESEARCH, vol. 12, no. 1, pages 1-8) refer to compositions comprising hyaluronic acid for the treatment of periodontitis. Prior art document A. Bitto (JOURNAL OF CLINICAL PERIODONTOLOGY, vol. 40, no. 1, pages 26-32) and JP 2018 197196 disclose compositions based on mixtures of polynucleotides for use in the treatment of periodontitis.

The present invention is based on the acknowledgement that co-administration of hyaluronic acid and polynucleotides, preferably via the topical and/or infiltration route, exerts a positive effect both on periodontitis and the regeneration of the interdental papilla, showing a synergy between the components which allows the achievement of results that are superior to the administration of the components used separately.

The present inventors found that, in the context of periodontitis and diseases characterised by recession of the interdental papilla, such as for example the black triangle syndrome, the combination of polynucleotides and hyaluronic acid has anti-inflammatory, trophic and biostimulant effects in the infiltration areas. It was also found that the composition for use according to the present invention, comprising the combination of polynucleotides and hyaluronic acid, wherein the hyaluronic acid is preferably not cross-linked, is quickly reabsorbed. Therefore, compared to the cross-linked hyaluronic acid infiltrations described in the prior art (see for example EP 1525011 and the aforementioned publications by Bertl et al., 2017), it is essential to acknowledge that, in this case, this is not a mere filling treatment.

Thanks to the biochemical characteristics of polynucleotides and hyaluronic acid, in fact, the present invention allows the achievement of an anti-inflammatory effect and a physiological response of the tissues, which is capable of promoting a remarkable and spontaneous regeneration of the interdental papilla and an improvement of tissue trophism.

Further advantages are that the administration of the composition for use according to the present invention via the topical and infiltration routes is well tolerated and not too unpleasant for the patient, even without the use of local anaesthesia; the treatment is rapid and non-invasive; the treatment is repeatable and without discomfort for the everyday life of the patient, who can immediately resume a normal social life and a normal diet, contrary to what happens instead after surgery; no side effects were reported; the treatment is extremely effective and therefore patients are highly satisfied.

Therefore, the object of the invention is a composition with a synergistic effect, comprising polynucleotides and hyaluronic acid as the active ingredients, and the use thereof in the applications defined by the appended claims, which form an integral part of the description.

Hyaluronic acid (HA) is a non-sulphur-containing polymer belonging to the glycosaminoglycan family. It is ubiquitous in mammalian tissues, has an unbranched structure and consists of the orderly repetition of a disaccharide composed of glucuronic acid and N-acetylglucosamine.

The size of the HA varies, but exceeds one million Daltons, ranging from 3 to 9 million Daltons in some tissues. Initially, HA was isolated in 1934 by Mayer from the hyaloid substance of the vitreous body, hence the name, and for years HA has been considered a filling and structural substance with extraordinary hydrophilic capacity, with no particular biological role. Only in the last decade, attention has focused on the biological role of this polymer, especially after the discovery of tissue receptors for HA, such as CD44 and RHAMM. The knowledge of the role of HA as an active substance in more complex processes concerning tissue regeneration and repair has therefore broadened. Therefore, HA is no longer considered to be an inert molecule, but a molecule actively involved in important biological processes, such as cell migration and duplication, angiogenesis, and tissue growth and maturation.

Within the scope of the present invention, hyaluronic acid can be used as such or be variously salified, for example with sodium.

Typically, hyaluronic acid or a sodium salt thereof is used with a molecular weight of between 500 and 3000 kDaltons, preferably between 600 and 1600 kDaltons. Further molecular weights of hyaluronic acid usable within the scope of the present invention are 700 kDaltons, 800 kDaltons, 900 kDaltons, 1000 kDaltons, 1200 kDaltons, 1400 kDaltons, 1600 kDaltons, 1800 kDaltons, 2000 kDaltons, 2200 kDaltons, 2400 kDaltons, 2600 kDaltons, 2800 kDaltons.

Non-crosslinked hyaluronic acid is preferably used.

The term "Polynucleotides" is intended to indicate a fraction of nucleic acid polymer chains, preferably DNA, having a molecular weight distribution preferably in the range between 0.5 kDaltons and 3000 kDaltons, more preferably between 30 kDaltons and 2000 kDaltons, with a numerical average molecular weight, for example, of approximately 250 kDaltons. Said fraction of DNA polymer chains is preferably obtained by extraction from animal or plant natural sources, such as fish sperm or plants, by means of a process which preferably comprises a step of partial fragmentation of the DNA chains, in order to reduce the molecular weights thereof, and a step of partial depurination of the DNA, to eliminate the informational capacity thereof.

The preferred percentage of DNA depurination is preferably in the range of from 1% to 5% purine bases removed (i.e. apurinic sites) out of the total purine bases originally present. A preferred depurination percentage is between 2% and 2.5%. Further depurination percentages of polynucleotides usable within the scope of the present invention are 1.5%, 1.6%, 1.7%, 1.8%, 1.9%, 2.1%, 2.2%, 2.3%, 2.4%, 2.6%, 2.7%, 2.8%, 2.9%, 3%, 3.1%, 3.2%, 3.3%, 3.4%, 3.5%, 3.6%, 3.7%, 3.8%, 3.9%, 4%, 4.1%, 4.2%, 4.3%, 4.4%, 4.5%, 4.6%, 4.7%, 4.8%, 4.9%.

From the foregoing, it is obvious that the characteristics of the PNs are significantly different from those of conventional polynucleotides which have not been subjected to:
- partial depurination,
- sterilisation techniques for viruses and prions,
- procedures for purification of the product which, when dry, exceeds 98% purity with quantities of proteins below 0.5% and a molecular weight range varying between 0.2 and 3000 kDaltons.

It is known and documented that PNs have the ability to induce mitosis in fibroblasts, endothelial cells and other cells. They have been used to stimulate tissue repair and in many experimental models have shown a remarkable ability to promote *ex novo* synthesis of extracellular matrix molecules.

In all wound-healing processes, the scientific literature confirmed that PNs have a positive effect on damage repair. PNs also have a remarkable ability to modulate the action of many inflammatory factors and interact with physiological growth factors, which makes these molecules very important and biologically very active in the growth of different cell lines and the secretory activity thereof.

The polynucleotides used in the present invention have a molecular weight distribution preferably in the range between 0.2 kDaltons and 3000 kDaltons, more preferably between 30 kDaltons and 2000 kDaltons, with a numerical average molecular weight of approximately 250 kDaltons, for example. The molecular weight distribution of the polynucleotides used in the scope of the present invention can also be included within the following ranges: 5-2500 kDaltons; 50-1500 kDaltons; 100-1000 kDaltons.

The composition according to the invention comprises:
- hyaluronic acid as described above
- polynucleotides (PNs) as described above.

For liquid formulations (for example formulations suitable for injection or topical application), for example, polyols are used in order to obtain a suitable osmolarity. A preferred polyol is mannitol in concentrations from 0 mg/ml to 50 mg/ml. To ensure an approximately neutral pH (7.0 +/- 1.0) a buffer system is used, preferably phosphate buffer.

The mannitol used can be sterile, pyrogen-free, pharmaceutical grade, for injectable use according to the European Pharmacopoeia.

The phosphate buffer used can be pharmaceutical grade, injectable, sterile, pyrogen-free.

In addition to liquid formulations, the PN + HA combination for use according to the present invention can also be used in the form of gel formulations known per se and commercially available, for example, as Nucliaskin^{®} Oral care from Mastelli S.r.l.

Compositions used typically comprise:
- polynucleotides from 1 mg/ml to 20 mg/ml, preferably from 5 mg/ml to 12 mg/ml,
- hyaluronic acid from 1 mg/ml to 20 mg/ml, preferably from 5 mg/ml to 12 mg/ml, with polynucleotide/hyaluronic acid weight ratios within the range from 0.2:1 to 2:1.

### Example 1

| | |
|---|---|
| Mannitol: | 40 mg/ml |
| phosphate buffer: | 1.3 mg/ml |
| hyaluronic acid: | 7.5 mg/ml |
| PNs: | 7.5 mg/ml |
| Water: | 943.7 mg/ml |

### Example 2

| | |
|---|---|
| Mannitol: | 40 mg/ml |
| phosphate buffer: | 1.3 mg/ml |
| hyaluronic acid: | 10 mg/ml |
| PNs | 10 mg/ml |
| Water: | 938.7 mg/ml |

### Example 3

| | |
|---|---|
| Mannitol: | 40 mg/ml |
| phosphate buffer: | 1.3 mg/ml |
| hyaluronic acid: | 3.5 mg/ml |
| PNs | 3.5 mg/ml |
| Water: | 951.7 mg/ml |

### Example 4

| | |
|---|---|
| Mannitol: | 40 mg/ml |
| phosphate buffer: | 1.3 mg/ml |
| hyaluronic acid: | 5 mg/ml |
| polynucleotides: | 15 mg/ml |
| Water: | 938.7 mg/ml |

### Example 5

| | |
|---|---|
| Mannitol: | 40 mg/ml |
| phosphate buffer: | 1.3 mg/ml |
| hyaluronic acid: | 3 mg/ml |
| PNs (respectively): | 7.5, 11, 16 mg/ml |
| Water: | 948.2; 944.7; 939.7 mg/ml |

### Example 6

| | |
|---|---|
| Mannitol: | 40 mg/ml |
| Phosphate buffer: | 1.3 mg/ml |
| Hyaluronic acid: | 17 mg/ml |
| Polynucleotides: | 3 mg/ml |
| Water: | 938.7 mg/ml |

The effectiveness of the compositions described above in the treatment of periodontitis has been determined by clinical trials on patients suffering from acute or chronic periodontitis. In these clinical trials, which are described in detail in the following experimental section, a commercial composition falling within the scope of the present invention (20 mg/ml Newest - class III medical device - CE 0373) was administered to the patients into the periodontal pockets and through local infiltration into the vestibule. The results obtained show that the administered composition was able to reduce the depth of the periodontal pocket in 94% of treated cases. A significant improvement was also observed in subjective parameters, such as burning, pain, discomfort, bleeding, swelling and redness.

The effectiveness of the compositions described above in the regeneration of the interdental papilla was determined by clinical trials on patients who exhibited at least one site with papilla loss in the aesthetic area, a papillary defect commonly referred to as the "black triangle syndrome". In these clinical trials, which are described in detail in the following experimental section, a commercial composition falling within the scope of the present invention (20 mg/ml Newest - class III medical device - CE 0373) was administered to the patients through infiltration into the papilla and the surrounding area (vestibule). The results obtained show that the administered composition was able to induce an improvement in the clinical picture of the papilla in 100% of treated cases. Furthermore, as shown by an extended clinical study, administration by infiltration of the aforementioned commercial composition did not cause the side or adverse effects described in the state of the art (in particular in Bertl K. et al., Clin Oral Implants Res. 2017 Sep;28(9):1054-1061(a) and Bertl K et al., Clin Oral Implants Res. 2017 Jul;28(7):871-876)(b)), presumably thanks to the combination of hyaluronic acid with polynucleotides.

The synergistic effect of the compositions comprising PNs and HA was determined by *in vitro* tests, described in detail in the following experimental section, aimed at determining the effect on the growth of human gingival fibroblast cells and on protein matrix deposition. The results obtained show that, while the effect of HA alone is modest, its combination with PNs results in increased effectiveness of the latter. It can therefore be concluded that by combining HA and PNs a synergistic effect is obtained at the gingival fibroblasts in terms of both cell regeneration and protein matrix deposition. This, in particular, may explain the special effectiveness of the HA and PN combination in the treatment of the black triangle syndrome and in periodontal therapies.

Lastly, studies were carried out for assessing local inflammation and the presence of metalloproteinases at periodontal pockets (assessed through the harvesting of crevicular fluid and measurement of alpha-2-macroglobulin and MMP-9 markers analysed by the ELISA method), in order to verify the modulation action of the product on inflammatory cytokines, as well as the local tolerability of a composition comprising the combination of PNs and HA.

In the study, 25 patients suffering from periodontal disease with at least 2 periodontal pockets were evaluated. All patients were treated with a normal hygiene and prophylaxis standard therapy, and in addition, the test MD was used in one of the two pockets, in a randomized way, through a single application in the periodontal pocket.

The results show that, in the case of alpha-2-macroglobulin, 100% of periodontal pockets treated with the test MD exhibited an improvement, that is a 68% improvement, whereas, in the untreated group, 1 pocket worsened, and the average improvement was 63%.

As for the MMP-9 parameter, the results show that periodontal pockets treated with the test MD exhibited a 69% improvement, whereas 6 untreated pockets worsened (24%) and the average improvement in the untreated pockets was significantly lower, that is 49%. These results are particularly significant also because each patient serves as a self-control, i.e., the patient has both a treated pocket and an untreated pocket, thus eliminating any possibility of interference due to individual variability.

In the laboratory, therefore with objective and blind parameters, it also appears that treatment with polynucleotides + hyaluronic acid brings about an improvement compared to the standard hygiene and prophylaxis therapy, in particular by modulating and reducing parameters related to inflammation and by reducing MMP9 activity. Also underlining the high tolerability and biocompatibility of the device.

### Experimental Section

### 1. Periodontal Therapy - Clinical Study

15 patients aged between 30 and 83 were treated in the study, including 2 men and 13 women suffering from acute periodontitis (3 patients) or chronic periodontitis (12 patients). Periodontitis is defined as periodontal tissue inflammation, which causes loss of attachment of the teeth to the alveoli, which results in formation of periodontal pockets, loose teeth, bleeding gums, abscesses and suppurations, ending in the loss of one or more teeth. 12/15 patients had disease familiarity when they entered the study, 6/15 were smokers. Each patient underwent hygiene and prophylaxis as per guidelines and was then treated with a polynucleotide and hyaluronic acid gel (20 mg/ml Newest - class III medical device - CE 0373).

**Table 1. Patients: general data**

| | |
|---|---|
| Total number of patients | 15 |
| age | min 30-max 83 |
| M/F | 2 M/13 F |
| acute periodontitis | 3 (out of 15) |
| chronic periodontitis | 12 (out of 15) |
| smokers | 6 (out of 15) |
| familiarity | 12 (out of 15) |

### Materials and Methods:

Each patient underwent a specific therapeutic program consisting of 3 main phases. One visit was scheduled for each phase, for a total of 3 visits. The program is described in detail below:
1)Pre-Treatment Phase: hygiene and prophylaxis sessions are scheduled and performed (for example, manual and ultrasonic therapy, aimed at decreasing the presence of plaque, tartar and therefore bacteria).

An average of 2-3 20-30-minute sessions of hygiene and prophylaxis were carried out for each patient on a monthly basis. Each patient underwent the first visit (V1) during this phase.

At V1, patients were clinically evaluated to record subjective parameters (symptoms reported by the patient) and objective parameters (signs detected by the physician).
2)Treatment phase with the test medical device: once clean, the patients were administered via the topical and infiltration routes with the polynucleotide and hyaluronic acid gel (20 mg/ml Newest); this phase was divided into several sessions:
- The first session involved topical application into the periodontal pockets and local infiltrations into the vestibule.
- The subsequent sessions, carried out on a monthly basis, only involved local infiltrations into the vestibule.

Each patient underwent the second visit (V2) during this phase. At V2, subjective and objective parameters were evaluated again.
3)Maintenance phase: a session of hygiene and local infiltrations into the vestibule with the polynucleotide and hyaluronic acid gel (20 mg/ml Newest) is carried out every 3-6 months.

Each patient underwent the last visit (V3) of the study during this phase. At V3, subjective and objective parameters were evaluated again, in order to evaluate the clinical course of the disease compared to the initial (basal) situation.

Application into the periodontal pockets was carried out by using a blunt tip applicator.

Local infiltrations into the vestibule were carried out with a 30G needle, at a distance of 0.5 -1 cm, after appropriate local disinfection.

### Results:

At the end of the study, 27% of treated cases exhibited a pocket depth reduction of 2 mm, whereas 67% of cases exhibited a reduction of 1 mm. Only in 6% of cases the pocket depth remained unchanged.

Burning, pain, discomfort, bleeding, swelling and redness were the subjective parameters assessed during the study at V1 (pre-treatment phase), V2 (treatment phase) and V3 (maintenance phase). All the tested parameters showed an improvement in the starting conditions. In detail:
Pain reduction, assessed by using a VAS scale with a score of 0-10, averaged 67.3%. In fact, the average value ranged from 3.7 at V1 to 1.2 at V3, with an average reduction of 2.5 points.

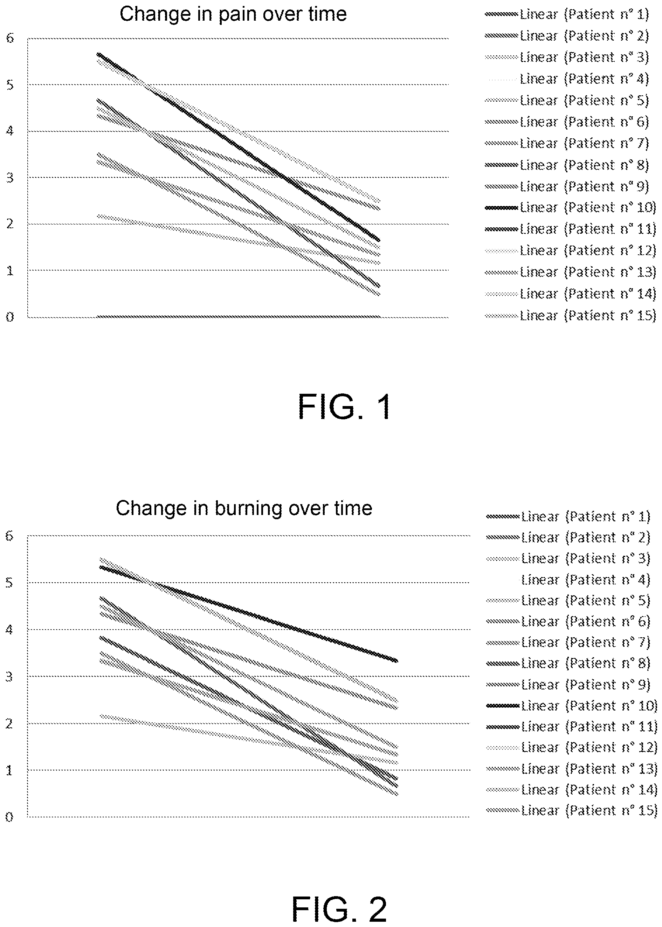

The graph in Figure 1 shows the reduction in the sensation of pain perceived by each patient from visit V1 to visit V3.

The reduction in burning, assessed by using a VAS scale with a score of 0-10, averaged 64.4%. In fact, the average value ranged from 3.9 at V1 to 1.4 at V3, with an average reduction of 2.5 points.

The graph in Figure 2 shows the reduction in the sensation of burning perceived by each patient from visit V1 to visit V3.

The reduction in discomfort, assessed by using a VAS scale with a score of 0-10, averaged 63.6%. In fact, the average value ranged from 3.7 at V1 to 1.3 at V3, with an average reduction of 2.3 points.

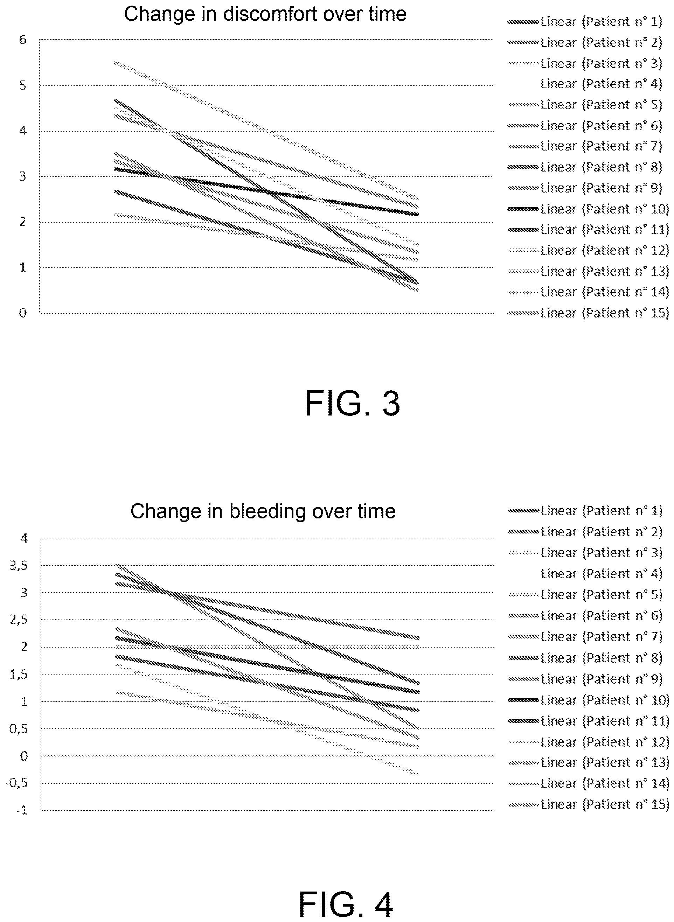

The graph in Figure 3 shows the reduction in the sensation of discomfort perceived by each patient from visit V1 to visit V3.

The reduction in bleeding, assessed on a scale with a score from 0-3, where 0 indicated no bleeding and 3 heavy bleeding, was on average 64.7%, in fact, the average value ranged from 2.3 at V1 to 0.8 at V3, with an average reduction of 1.5 points.

The graph in Figure 4 shows the reduction in bleeding obtained for each patient from visit V1 to visit V3.

In conclusion, the treatment of chronic and generalized periodontal diseases (11/15 cases treated) with the gel containing the PN + HA combination gave excellent results in terms of disappearance of swelling and redness. There was also an improvement of over 60% in signs such as bleeding and symptoms such as burning, pain and discomfort. There was also an objective improvement in terms of reduction of the pocket depth.

Therefore, in the dental field, treatment with the gel containing the PN + HA combination represents a valuable and innovative therapeutic choice associated with hygiene and prophylaxis sessions, to improve and enhance the response to the classic hygiene and prophylaxis therapy.

### 2. Clinical study of papilla regeneration (black triangle syndrome)

10 patients with an average age of 52 years, including 7 men and 3 women, were treated in the study. Two of the patients at the start of the study were smokers. In all cases, the patients exhibited at least one site with papilla loss in the aesthetic area, a papillary defect commonly referred to as the "black triangle syndrome".

**Table 2. Patients: general data**

| | |
|---|---|
| Total number of patients | 10 |
| average age | 52 |
| M/F | 7 M/3 F |
| smokers | 2 (out of 10) |

### Materials and Methods:

Each patient underwent a specific therapeutic program consisting of 2 main phases. One visit was scheduled for each phase during the study and one follow-up visit was scheduled after the treatment phase, for a total of 3 visits. The program is described in detail below:
1)Pre-Treatment Phase: The phase in which hygiene and prophylaxis sessions were scheduled and performed (for example, manual and ultrasonic therapy, aimed at decreasing the presence of plaque, tartar and therefore bacteria). All patients were instructed to perform mechanical cleaning at home by using an ultra-soft toothbrush and a brushing technique with movements in the apical-coronal direction.

An average of 2-3 20-30-minute sessions of hygiene and prophylaxis were carried out for each patient on a monthly basis. Each patient underwent the first visit (V1) during this phase.

Photographs were taken at V1 and the physician's clinical evaluation and patient satisfaction were recorded.
2)Treatment phase with the test medical device: At least one month after stabilization of the clinical picture following the hygiene and prophylaxis sessions, the patients were administered with the polynucleotide and hyaluronic acid gel (20 mg/ml Newest).

After disinfection with mouth rinses and local disinfectant at the infiltration site, the affected area was treated by infiltrating the papilla and the surrounding area (vestibule) with small doses of product, generally less than 0.1-0.2 ml. Local infiltrations into the vestibule were carried out with a 30G needle, at a distance of 0.5 -1 cm.

The treatment was repeated three to six weeks apart for a total of 3-10 infiltrations.

Each patient underwent the second visit (V2) during this phase. At V2, the effects were documented with photographs, compared with those taken before treatment and by filling in a data collection form with recording of the physician's clinical evaluation and patient satisfaction.

### Results:

Patients' subjective evaluations were very positive both from the point of view of pain and compliance with the treatment and of satisfaction with said treatment, with the following results.

The clinical results were very encouraging. Evaluation based on a quartile scale showed improvement in 100% of patients. All cases showed a clinical improvement of the papilla, although not all of them showed complete healing.

On average, regrowth started to appear after the second or third session, although in some cases regrowth began after the seventh or eighth session.

As regards the objective evaluation of the papillae, all patients showed improvement in the papillary structure, such as to change their classification:
- in three cases, class III papilla became class I papilla
- in six cases, class II papilla became class I papilla
- in one case, class I papilla became a Normal papilla (the papilla fills the interdental space again up to the point of contact).

In particular, it should be underlined that the improvement of the papilla occurred very quickly: all patients already exhibited an initial regeneration of the papilla after one week and this regenerative process continued over time. In fact, the sites were checked in a follow-up and the improvement that took place after the applications, one month after the last treatment, was still present. In no case was there a recurrence.

In conclusion, a trophic/regenerative effect of polynucleotides, which were only locally infiltrated, was shown for the first time in the treatment of the black triangle syndrome, a disease hitherto treated with invasive methods such as orthodontic, restorative and surgical interventions, which however yielded poor clinical results.

### 3. In vitro study on gingival fibroblasts

Human gingival fibroblasts were cultured on growth medium (Dulbecco's Modified Eagle Medium) supplemented with 10% bovine foetal serum (BFS). Cells were seeded at a density of 10,000/cm². 24 hours after seeding the cells were incubated under the conditions shown in Table 3, again prepared in DMEM + 10% BFS.

Cells were counted and proteins were quantified after 3 days of treatment.

The number of cells was measured with a particle counter. Proteins were quantified by spectrophotometric reading of stained protein lysates by using a modification of Lowry's method.

Table 3 shows the concentrations of the tested compositions and the values relating to cell growth and protein content, expressed as % of the control. The control is the fibroblast cell culture not subjected to any treatment, measured at the same time point.

**Table 3**

| **Product** | **% gingival fibroblasts vs control: cell growth** | **% gingival fibroblasts vs control: proteins** |
|---|---|---|
| pn 50 µg/ml | 123 | 124 |
| pn 100 µg/ml | 127 | 129 |
| pn 300 µg/ml | 128 | 129 |
| ha 50 µg/ml | 102 | 103 |
| ha 100 µg/ml | 105 | 104 |
| ha 300 µg/ml | 106 | 104 |
| pn+ha 50 µg/ml | 127 | 130 |
| pn+ha 100 µg/ml | 131 | 130 |
| pn+ha 300 µg/ml | 132 | 133 |
| control | 100 | 100 |

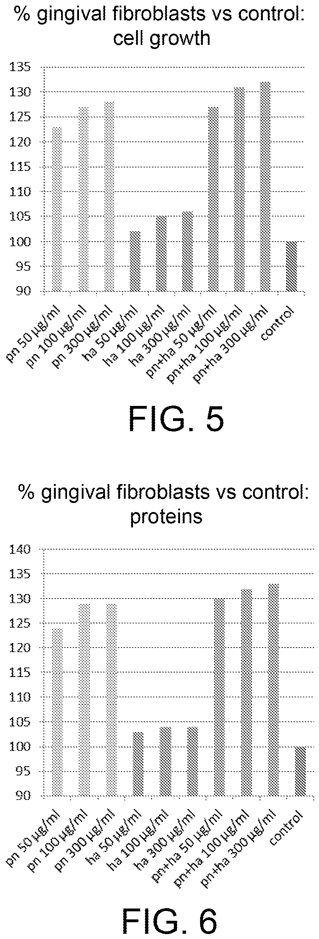

Cell growth and protein quantification results are also shown in figures 5 and 6.

The results show that hyaluronic acid does not change its action as its concentration increases, constantly maintaining a mild effect both in terms of protein expression and cell growth. Instead, it is clear that polynucleotides increase their effect as the dose increases. Compared to the individual components, the effect obtained with the mixture - which, for each total concentration, consisted of 50% hyaluronic acid and 50% polynucleotides - was consistently superior to the effect of the PNs used alone and the HA used alone.

Accordingly, bearing in mind the low effect of hyaluronic acid alone, it is obvious that the presence of the latter is important for increasing the effect of the PNs, which are the most active component in cell growth and protein expression. It can be concluded that by combining HA and PNs a synergistic effect is obtained at the gingival fibroblasts in terms of both cell regeneration and protein matrix deposition. This, in particular, may explain the special effectiveness of the HA and PN combination in the treatment of the black triangle syndrome and in periodontal therapies.

### 4. In two study of the crevicular fluid

Treatment of periodontitis first of all involves controlling bacteria and inflammation, and immediately afterwards, the subsequent step of regenerating the lost, hard and soft periodontal tissues becomes essential, in order to restore tooth attachment and eliminate gingival and bone pockets.

ODONTO-PNHA is a class III Medical Device manufactured by Mastelli Srl - Sanremo (IM). It is an original combination of polynucleotides and hyaluronic acid for application in periodontal pockets.

The main purpose of the clinical study with Odonto -PNHA is to evaluate the efficacy and safety of a medical device in non-surgical periodontal therapy. The experimental hypothesis is that the addition of ODONTO-PNHA can help obtain periodontal attachment gain in the periodontal pockets, in order to avoid a surgical procedure.

To evaluate this, a total of 50 patients were recruited: each patient provided a test site (treated with ODONTO-PNHA) and a control site (untreated). Patients will be followed up for 12 months after treatment. The study is performed in a single clinical centre, at the Department of Periodontology, Università La Sapienza.

Primary objective: evaluation of ODONTO-PNHA's performance on Probing Pocket Depth (PPD), Marginal Bleeding Index (MB), Modified Plaque Index (mPLI), pain, Investigator Global Satisfaction with the usability of the device and Patient Global Satisfaction with the performance of the device (5-point Likert scale). Tolerability and safety assessment.

Objective of the biochemical analysis: Evaluation of the effects of ODONTO-PNHA on the inflammatory state of the tissues around the tooth in 25 subjects. This was assessed through harvesting the crevicular fluid and assessing alpha 2 macroglobulin (α2M) and metalloprotease-9 (MMP-9) in the fluid at baseline and at week 6.

The crevicular fluid samples, adsorbed on methylcellulose strips, were processed and assayed with the ELISA method.

Matrix metalloproteinases are an important group of proteinases related to collagen degradation during the destructive process of periodontal disease, which can be measured in the gingival crevicular fluid. Alpha 2 macroglobulins are plasma proteins that participate in the regulation of blood clotting and immune response and increase in the presence of a chronic infection. 25 patients suffering from periodontal disease and recruited in the ODONTO-PNHA-01 clinical study underwent two takings of crevicular fluid, one taking from the periodontal pocket treated with the test MD and one from an untreated pocket.

The takings were performed at baseline (Visit 1) and at week 6 (Visit 2). The total 4 samples were assessed for the presence and concentration of inflammation markers.

### Results

After 6 weeks, the concentrations of the markers, both in the pocket treated with the polynucleotide and hyaluronic acid MD and in the untreated pocket, were significantly lower compared to week 0, showing a general improvement in periodontal disease, thanks to the standard hygiene and prophylaxis therapy.

Analysis of data from the test MD-treated and untreated periodontal pockets showed a further difference, described below, to the advantage of patients who had received treatment with the test MD.

In the treated pockets, the average alpha 2 macroglobulin value was 1898.71 at time 0 and 605.90 after 6 weeks, with an average reduction of 1292.81 corresponding to 68.09%. Out of 25 treated pockets, 19 (76%) showed an improvement of more than 50% (Table 4).

In the untreated pockets, the average alpha 2 macroglobulin value was 1665 at time 0 and 601 after 6 weeks, with an average reduction of 1064 corresponding to 63.91%. Out of 25 treated pockets, 17 (68%) showed an improvement of more than 50% (Table 4).

**Table 4:**

| | TREATED | CONTROLS |
|---|---|---|
| Total number of patients | 25 | 25 |
| Start of the study | 1898.7 | 1665.3 |
| 6 weeks | 605.9 | 601.1 |
| Delta | 1292.8 | 1064.3 |
| % Improvement compared to baseline | 68.1% | 63.9% |
| No. improved patients> 50% | 19 | 17 |

An even greater difference between treated and controls was found in the MMP-9 parameter. In the treated pockets, the average MMP-9 value was 234.9 at time 0 and 72.9 after 6 weeks, with an average reduction of 162.00 corresponding to 68.96%. Out of 25 treated pockets, 18 (72%) showed an improvement of more than 50% (Table 5).

In the untreated pockets, the average MMP-9 value was 173.4 at time 0 and 87.4 after 6 weeks, with an average reduction of 86 corresponding to 49.58%. Out of 25 treated pockets, only 9 (36%) showed an improvement of more than 50% (Table 5).

**Table 5:**

| | TREATED | CONTROLS |
|---|---|---|
| Total number of patients | 25 | 25 |
| Start of the study | 234.9 | 173.4 |
| 6 weeks | 72.9 | 87.4 |
| Delta | 162.0 | 86.0 |
| % Improvement compared to baseline | 68.96% | 49.58% |
| No. improved patients> 50% | 18 out of 25 (72%) | 9 out of 25 (36%) |
| No. worsened patients | 2 (8%) | 6 (24%) |

The results show that, in the case of alpha 2 macroglobulin, all treated patients showed an improvement, whereas in the untreated group, 1 patient worsened; in the case of the MMP-9 parameter, all patients except 1 showed an improvement, whereas in the untreated group, 6 patients showed a worsening.

In general, improvement was greater in the test MD-treated patients and this improvement was particularly clear for the MMP-9 parameter.

These results are particularly significant also because each patient serves as a self-control, i.e., the patient has both a treated pocket and an untreated pocket, thus eliminating any possibility of interference due to individual variability.

### Conclusion

In the laboratory, therefore with objective and blind parameters, it also appears that treatment with polynucleotides + hyaluronic acid brings about an improvement compared to the standard hygiene and prophylaxis therapy, in particular by modulating and reducing parameters related to inflammation and by reducing MMP9 activity.

Therefore, the preliminary laboratory results illustrated above support the experimental hypothesis that the PN + HA combination can help obtain periodontal attachment gain in periodontal pockets, in order to avoid surgical procedure.

## Claims

1. A composition comprising hyaluronic acid and a mixture of polynucleotides extracted from a natural source, wherein said mixture of polynucleotides has a depurination ratio ranging from 1% to 5% purine bases removed out of the total purine bases originally present and it is a fraction of nucleic acid polymer chains having a molecular weight distribution ranging from 0.5 kDaltons to 3,000 kDaltons for use as an active substance in the treatment for regeneration of the interdental papilla or in the treatment of the recession of the interdental papilla (black triangle syndrome) or in the treatment of periodontitis

2. The composition for use according to claim 1, wherein said periodontitis is acute periodontitis or chronic periodontitis.

3. The composition for use according to claim 1 or 2, wherein said treatment results in reduction in the depth of the periodontal pockets and/or in pain reduction and/or in stinging reduction and/or in discomfort reduction and/or in bleeding reduction.

4. The composition for use according to any one of claims 1 to 3, wherein said treatment causes the regeneration of the papilla.

5. The composition for use according to any one of claims 1 to 4, wherein said treatment results in increased proliferation of gingival fibroblasts and increased deposition of a protein matrix.

6. The composition for use according to any one of claims 1 to 5, wherein the treatment is carried out by topical application and/or local infiltration.

7. The composition for use according to any one of claims 1 to 6, wherein the treatment is carried out by topical application in the periodontal pockets.

8. The composition for use according to any one of claims 1 to 7, wherein the treatment is carried out by local gingival infiltration in the papilla and/or in the vestibule.

9. The composition for use according to any one of claims 1 to 8, comprising from 1 mg/ml to 20 mg/ml of hyaluronic acid and from 1 mg/ml to 20 mg/ml of said mixture of polynucleotides.

10. The composition for use according to claim 9, comprising from 5 mg/ml to 12 mg/ml of hyaluronic acid and from 5 mg/ml to 12 mg/ml of said mixture of polynucleotides.

11. The composition for use according to claims 9 or 10, comprising the polynucleotides and the hyaluronic acid in a weight ratio of from 0.2:1 to 2:1.

12. The composition for use according to any one of claims 1 to 11, wherein said mixture of polynucleotides is a fraction of nucleic acid polymer chains having a molecular weight distribution ranging from 30 kDaltons to 2,000 kDaltons, for example with an average molecular weight of approximately 250 kDaltons.

13. The composition for use according to any one of claims 1 to 12, wherein said mixture of polynucleotides is obtainable by extraction from a natural, animal or plant source, by a process comprising a step of partial fragmentation of the polynucleotide nucleic acid chains and a step of partial depurination of the nucleic acid.

14. The composition for use according to any one of claims 1 to 13, wherein the hyaluronic acid is not cross-linked.

15. The composition for use according to any one of claims 1 to 14, comprising hyaluronic acid with a molecular weight of between 500 kDaltons and 3,000 kDaltons.

16. The composition for use according to any one of claims 1 to 15 in the liquid form, free of physiological saline solution, in an aqueous solvent comprising a pharmaceutically acceptable polyol, preferably mannitol.

17. The composition for use according to claim 16, having an osmolarity between 10 m.o.s./l and 500 m.o.s./l, preferably between 250 m.o.s./l and 350 m.o.s./l.

18. The composition for use according to any one of claims 1 to 17, in a dosage form suitable for topical application or local infiltration.

## Patentansprüche

1. Zusammensetzung, umfassend Hyaluronsäure und eine Mischung aus Polynukleotiden, die aus einer natürlichen Quelle extrahiert wurden, wobei die Mischung aus Polynukleotiden eine Depurinierungsrate im Bereich von 1% bis 5% der Purinbasen aufweist, die von allen ursprünglich vorhandenen Purinbasen entfernt werden, und eine Fraktion er Nukleinsäure-Polymerketten mit einer Molekulargewichtsverteilung im Bereich von 0,5 kDalton bis 3000 kDalton zur Verwendung als Wirkstoff bei der Regenerationsbehandlung für die Interdentalpapille oder bei der Behandlung der Rezession der Interdentalpapille (Black-Dreieck-Syndrom) oder bei der Behandlung von Parodontitis darstellt.

2. Zusammensetzung zur Verwendung nach Anspruch 1, wobei es sich bei der Parodontitis um eine akute Parodontitis oder eine chronische Parodontitis handelt.

3. Zusammensetzung zur Verwendung nach Anspruch 1 oder 2, wobei die Behandlung zu einer Verringerung der Tiefe parodontaler Taschen und/oder einer Verringerung der Schmerzen und/oder einer Verringerung des Kribbelns und/oder einer Verringerung der Beschwerden und/oder einer Verringerung der Blutung führt.

4. Zusammensetzung zur Verwendung nach einem der Ansprüche 1 bis 3, wobei die Behandlung die Regeneration der Papille bewirkt.

5. Zusammensetzung zur Verwendung nach einem der Ansprüche 1 bis 4, wobei die Behandlung zu einer erhöhten Proliferation gingivaler Fibroblasten und einer erhöhten Ablagerung einer Proteinmatrix führt.

6. Zusammensetzung zur Verwendung nach einem der Ansprüche 1 bis 5, wobei die Behandlung durch topische Anwendung und/oder lokale Infiltration erfolgt.

7. Zusammensetzung zur Verwendung nach einem der Ansprüche 1 bis 6, wobei die Behandlung durch topische Anwendung in den Parodontaltaschen erfolgt.

8. Zusammensetzung zur Verwendung nach einem der Ansprüche 1 bis 7, wobei die Behandlung durch lokale gingivale Infiltration in der Papille und/oder im Vestibulum erfolgt.

9. Zusammensetzung zur Verwendung nach einem der Ansprüche 1 bis 8, umfassend 1mg/ml bis 20mg/ml Hyaluronsäure und 1mg/ml bis 20mg/ml der Polynukleotidmischung.

10. Zusammensetzung zur Verwendung nach Anspruch 9, umfassend 5mg/ml bis 12mg/ml Hyaluronsäure und 5mg/ml bis 12mg/ml der Polynukleotidmischung.

11. Zusammensetzung zur Verwendung nach Anspruch 9 oder 10, umfassend Polynukleotide und Hyaluronsäure in einem Gewichtsverhältnis von 0,2:1 bis 2:1.

12. Zusammensetzung zur Verwendung nach einem der Ansprüche 1 bis 11, wobei die Polynukleotidmischung eine Fraktion von Nukleinsäure-Polymerketten mit einer Molekulargewichtsverteilung im Bereich von 30 kDalton bis 2000 kDalton, beispielsweise mit einem durchschnittlichen Molekulargewicht von ungefähr 250 kDalton, darstellt.

13. Zusammensetzung zur Verwendung nach einem der Ansprüche 1 bis 12, wobei das Polynukleotidgemisch durch Extraktion aus einer natürlichen, tierischen oder pflanzlichen Quelle, durch ein Verfahren, das einen Schritt der teilweisen Fragmentierung der Polynukleotid-Nukleinsäureketten und einen Schritt der teilweisen Depurinierung der Nukleinsäure umfasst, erhältlich ist.

14. Zusammensetzung zur Verwendung nach einem der Ansprüche 1 bis 13, wobei die Hyaluronsäure nicht vernetzt ist.

15. Zusammensetzung zur Verwendung nach einem der Ansprüche 1 bis 14, umfassend Hyaluronsäure mit einem Molekulargewicht zwischen 500 kDalton und 3000 kDalton.

16. Zusammensetzung zur Verwendung nach einem der Ansprüche 1 bis 15 in flüssiger Form, frei von physiologischer Salzlösung, in einem wässrigen Lösungsmittel, das ein pharmazeutisch verträgliches Polyol, vorzugsweise Mannitol, umfasst.

17. Zusammensetzung zur Verwendung nach Anspruch 16 mit einer Osmolalität zwischen 10m.o.s./l und 500m.o.s./l, vorzugsweise zwischen 250m.o.s./l und 350m.o.s./l.

18. Zusammensetzung zur Verwendung nach einem der Ansprüche 1 bis 17 in einer Arzneiform, die zur topischen Anwendung oder lokalen Infiltration geeignet ist.

## Revendications

1. Composition comprenant de l'acide hyaluronique et un mélange de polynucléotides extraits d'une source naturelle, ledit mélange de polynucléotides présentant un taux de dépurination dans la gamme de 1 % à 5 % de bases puriques éliminées de la totalité des bases puriques initialement présentes et étant une fraction de chaînes polymères d'acide nucléique ayant une distribution de poids moléculaire dans la gamme de 0,5 kDaltons à 3000 kDaltons afin d'être utilisée comme substance active dans le traitement régénératif de la papille interdentaire ou dans le traitement de la récession de la papille interdentaire (syndrome du triangle noir) ou dans le traitement de la parodontite.

2. Composition destinée à être utilisée selon la revendication 1, ladite parodontite étant une parodontite aiguë ou une parodontite chronique.

3. Composition destinée à être utilisée selon la revendication 1 ou 2, ledit traitement entraînant une réduction de la profondeur des poches parodontales et/ou une réduction de la douleur et/ou une réduction des picotements et/ou une réduction de l'inconfort et/ou une réduction des saignements.

4. Composition destinée à être utilisée selon l'une quelconque des revendications 1 à 3, ledit traitement provoquant la régénération de la papille.

5. Composition destinée à être utilisée selon l'une quelconque des revendications 1 à 4, ledit traitement entraînant une prolifération accrue de fibroblastes gingivaux et un dépôt accru d'une matrice protéique.

6. Composition destinée à être utilisée selon l'une quelconque des revendications 1 à 5, le traitement étant réalisé par application topique et/ou infiltration locale.

7. Composition destinée à être utilisée selon l'une quelconque des revendications 1 à 6, le traitement étant réalisé par application topique dans les poches parodontales.

8. Composition destinée à être utilisée selon l'une quelconque des revendications 1 à 7, le traitement étant réalisé par infiltration gingivale locale dans la papille et/ou dans le vestibule.

9. Composition destinée à être utilisée selon l'une quelconque des revendications 1 à 8, comprenant de 1 mg/ml à 20 mg/ml d'acide hyaluronique et de 1 mg/ml à 20 mg/ml dudit mélange de polynucléotides.

10. Composition destinée à être utilisée selon la revendication 9, comprenant de 5 mg/ml à 12 mg/ml d'acide hyaluronique et de 5 mg/ml à 12 mg/ml dudit mélange de polynucléotides.

11. Composition destinée à être utilisée selon les revendications 9 ou 10, comprenant les polynucléotides et l'acide hyaluronique dans un rapport n poids de 0,2:1 à 2:1.

12. Composition destinée à être utilisée selon l'une quelconque des revendications 1 à 11, ledit mélange de polynucléotides étant une fraction de chaînes polymères d'acide nucléique ayant une distribution de poids moléculaire dans la gamme de 30 kDaltons à 2000 kDaltons, par exemple avec un poids moléculaire moyen d'environ 250 kDaltons.

13. Composition destinée à être utilisée selon l'une quelconque des revendications 1 à 12, ledit mélange de polynucléotides pouvant être obtenu par extraction d'une source naturelle, animale ou végétale, par un processus comprenant une étape de fragmentation partielle des chaînes d'acide nucléique des polynucléotides et une étape de dépurination partielle de l'acide nucléique.

14. Composition destinée à être utilisée selon l'une quelconque des revendications 1 à 13, l'acide hyaluronique n'étant pas réticulé.

15. Composition destinée à être utilisée selon l'une quelconque des revendications 1 à 14, comprenant de l'acide hyaluronique de poids moléculaire compris entre 500 kDaltons et 3000 kDaltons.

16. Composition destinée à être utilisée selon l'une quelconque des revendications 1 à 15 sous forme liquide, exempte de solution saline physiologique, dans un solvant aqueux comprenant un polyol acceptable du point de vue pharmaceutique, de préférence du mannitol.

17. Composition destinée à être utilisée selon la revendication 16, présentant une osmolalité comprise entre 10 m.o.s./l et 500 m.o.s./l, de préférence entre 250 m.o.s./l et 350 m.o.s./l.

18. Composition destinée à être utilisée selon l'une quelconque des revendications 1 à 17, sous une forme galénique adaptée à une application topique ou à une infiltration locale.
